# EUROPÄISCHE PATENTSCHRIFT

(11) **EP 1 746 421 B1**
(45) Veröffentlichungstag und Bekanntmachung des Hinweises auf die Patenterteilung: **30.07.2008**
(21) Anmeldenummer: 06021523.3
(22) Anmeldetag: 13.04.2000
(51) Int. Cl.: G01N 33/574, G01N 33/577, G01N 33/68, A61K 39/395, A61K 47/48, A61K 51/10, C12N 15/13, C07K 16/28, C07K 19/00

(54) **Diagnostischer und therapeutischer Einsatz von Antikörpern gegen den Urokinase-Rezeptor**
Diagnostic and therapeutic application of antibodies against the urokinase receptor
Utilisation thérapeutique et diagnostique d'anticorps dirigés contre le récepteur d'urokinase

(30) Priorität: 13.04.1999 EP 99107199
(43) Veröffentlichungstag der Anmeldung: 24.01.2007
(62) Teilanmeldung aus: 00917083.8
(73) Patentinhaber: Wilex AG, 81675 München (DE)
(72) Erfinder: Schmitt, Manfred, 81669 München (DE); Noack, Frank, 23558 Lübeck (DE); Graeff, Heinrich, 81479 München (DE); Harbeck, Nadia, 83624 Otterfing (DE)
(74) Vertreter: Weiss, Wolfgang

(56) Entgegenhaltungen:
- EP-A- 0 317 156
- US-A- 5 234 816
- W. XUE ET AL.: "Urokinase-type plasminogen activator receptors associate with beta1 and beta3 integrins of fibrosarcoma cells: dependence on extracellular matrix components" CANCER RESEARCH, Bd. 57, Nr. 9, 1. Mai 1997 (1997-05-01), Seiten 1682-1689, XP002117246
- H. ALLGAYER ET AL.: "Immunocytochemical phenotyping of disseminated tumor cells in bone marrow by uPA receptor and CK18: investigation of sensitivity and specificity of an immunogold/alkaline phosphatase double staining protocol" JOURNAL OF HISTOCHEMISTRY AND CYTOCHEMISTRY, Bd. 45, Nr. 2, Februar 1997 (1997-02), Seiten 203-212, XP002117247
- M. HEISS ET AL.: "Individual development and uPA-receptor expression of disseminated tumour cells in bone marrow: a reference to early systemic disease in solid cancer" NATURE MEDICINE, Bd. 1, Nr. 10, Oktober 1995 (1995-10), Seiten 1035-1039, XP002117248

## Beschreibung

Die Erfindung betrifft ein Verfahren und einen Reagenzienkit zum Nachweis von Zellen in einer biologischen Probe in vitro unter Verwendung einer Doppelfluoreszenztechnik.

Der zuverlässige Nachweis von verstreuten Tumorzellen, die aus dem soliden Gewebeverband ausgebrochen sind (Mikrometastasen), ist für die Tumordiagnostik und Therapie von großer Bedeutung. Im Laufe der vergangenen Jahre wurden deshalb verschiedene Verfahren entwickelt, um solche einzelnen verstreuten Tumorzellen in Körperflüssigkeiten oder Gewebeproben nachzuweisen. Der Nachweis kann z.B. durch selektive Markierung der seltenen Zellen mittels immunzytochemischer Methoden erfolgen, wobei häufig enzymatische Markierungsgruppen wie Alkalische Phosphatase eingesetzt werden. Auch Doppelmarkierungstechniken sind bekannt.

Die Veröffentlichung Schlimok et al. (Proc. Natl. Acad. Sci. USA 84 (1987) 8672-8676) beschreibt den Nachweis von mikrometastatischen Tumorzellen in Knochenmark mittels einer Doppelmarkierungstechnik, wobei ein für Zellen epidermalen Ursprungs spezifischer Cytokeratin 18-Antikörper und ein Leukozyten-Antikörper verwendet werden. Dabei werden Alkalische Phosphatase und eine radioaktive Markierungsgruppe (¹²⁵I) eingesetzt. Da die Verwendung radioaktiver Markierungsgruppen mit Nachteilen behaftet ist, eignet sich diese Methode nicht für die klinische Praxis.

Funke et al. (Int. J. Cancer 65 (1996), 755-761) beschreiben den Nachweis von Mikrometastasen in Knochenmark mittels einer Doppelmarkierungstechnik unter Verwendung eines Cytokeratin 18-Antikörpers und eines E-Cadherin-Antikörpers. Beide Antikörper werden über Alkalische Phosphatase als enzymatische Markierungsgruppe und zwei unterschiedlich gefärbte chromogene Substrate nachgewiesen. Der sequenzielle Nachweis beider Antikörper mittels unterschiedlicher chromogener Substrate ist jedoch umständlich und deshalb für die klinische Praxis wenig geeignet.

Heiss und Mitarbeiter (Heiss et al., Nature Med. 1 (1995), 1035-1039 und Allgayer et al., J. Histochem. Cytochem. 45 (1997), 203-212) weisen verstreute Tumorzellen in Knochenmark über eine Doppelmarkierungsmethode basierend auf dem gleichzeitigen Nachweis von Cytokeratin 18 und dem uPA-Rezeptor (uPAR) nach. Hierzu werden auf einem Objektträger gebundene, fixierte Zellen mit einem biotinylierten Cytokeratin-spezifischen Antikörper und anschließend mit einem Konjugat aus Alkalischer Phosphatase und Streptavidin inkubiert. Mittels der immobilisierten Alkalischen Phosphatase und einem chromogenen Substrat wird eine enzymatische Färbereaktion durchgeführt, wobei eine dunkelrote Färbung entsteht. Zusätzlich wird ein monoklonaler Antikörper gegen uPAR eingesetzt, der mit einem goldkonjugierten Sekundärantikörper markiert und anschließend einer Silber-Verstärkungsreaktion unterzogen wird, wobei eine schwarze Färbung resultiert. Die Objektträger werden dann auf die Färbungen (dunkelrot/schwarz) manuell und visuell unter dem Mikroskop durchgemustert, wobei eine Doppelfärbung extrem schwer zu detektieren ist.

Die Veröffentlichung Xue et al. (Cancer Research, 57 (1997), 1682-1689) beschreibt eine Studie über die physikalische Wechselwirkung zwischen dem Urokinase-Plasminogen-Aktivator-Rezeptor (uPAR) und den Integrinen β₁ und β₃, abhängig von der Anhaftung der Tumorzellen an unterschiedlichen Bestandteilen der extrazellulären Matrix. Bei diesen Untersuchungen wird ein Doppelfluoreszenzverfahren beschrieben, wobei ein für den Urokinaserezeptor spezifisches Bindemolekül und ein für die Integrine β₁ und β₃ spezifisches Bindemolekül verwendet wird. Dabei ist der jeweilige Integrin-spezifische Antikörper direkt fluoreszenzmarkiert, wohingegen der Urokinaserezeptor-spezifische Antikörper indirekt über einen an ein sekundäres Bindemolekül konjugierten Fluoreszenzfarbstoff nachgewiesen wird.

EP-A-0 317 156 beschreibt den Nachweis von weißen Blutzellen aus Knochenmark- und Blutproben unter Verwendung eines Doppelfluoreszenzprotokolls, wobei zwei direkt fluoreszenzmarkierte Antikörper spezifisch für Moleküle auf der Oberfläche der zu markierenden Zellen verwendet werden.

Die der vorliegenden Erfindung zugrundeliegende Aufgabe bestand darin, ein in vitro Verfahren zum Nachweis von Zellen, insbesondere von selten vorkommenden Zellen wie Tumorzellen in einer biologischen Probe, z.B. Knochenmark, durchzuführen, bei dem die Nachteile des Standes der

Technik mindestens teilweise beseitigt sind. Insbesondere soll das Verfahren gleichzeitig eine hohe Sensitivität und eine problemlose Auswertbarkeit ermöglichen.

Diese Aufgabe wird gelöst durch ein in vitro Verfahren zum Nachweis von Zellen in einer biologischen Probe, das folgende Schritte umfaßt:
(a) Inkontaktbringen einer zu testenden Probe mit mindestens zwei verschiedenen, die nachzuweisenden Zellen erkennenden Bindemolekülen, wobei die Bindemoleküle mit jeweils verschiedenen Fluoreszenzfarbstoffen markiert werden, und
(b) Bestimmen der Fluoreszenzmarkierungen in der auf einer Festphase fixierten Probe.

Das erfindungsgemäße Verfahren eignet sich zum Nachweis selten vorkommender Zellen in einer fixierten biologischen Probe. "Selten vorkommend" im Sinne der vorliegenden Erfindung bedeutet dabei, daß die erwartete Häufigkeit der nachzuweisenden Zellen im Bereich von 1:10⁴ bis 1:10⁷ der gesamten in der zu testenden Probe vorhandenen Zellen ist. Beispiele für solche selten vorkommenden Zellen sind Tumorzellen in einer Blut- oder Knochenmarksprobe. Bei entsprechender Auswahl von zellspezifischen Determinanten und dagegen gerichteten Bindemolekülen können selbstverständlich auch andere Arten selten vorkommender Zellen nachgewiesen werden.

Das erfindungsgemäße Verfahren ermöglicht aufgrund der Doppelfluoreszenz-Färbetechnik eine schnelle und genaue Identifizierung der nachzuweisenden Zellen. Außerdem können bei Verwendung unterschiedlicher, vorzugsweise nebeneinander nachweisbarer Fluoreszenzmarkierungen in sowie auf der Zelle kolokalisierte Antigene (wie z.B. Cytokeratin 8/18, p53, PAI-2 und insbesondere der Urokinase-Rezeptor uPAR) analysiert werden. Dies ist bisher mit bekannten Methoden insbesondere bei Gewebeproben wie Knochenmarksaspiraten sehr schwierig gewesen. Ein weiterer großer Vorteil der neu entwickelten Methode ist die Möglichkeit, die Anzahl und Intensität fluoreszierender Zellen quantitativ zu bestimmen, beispielsweise mit einem konfokalen Laser-Scanning-Mikroskop.

Zur Bereitstellung einer zu testenden biologischen Probe kann eine Probe dem Patienten, z.B. aus einer Körperflüssigkeit wie etwa Blut oder aus einem Gewebe wie etwa Knochenmark entnommen werden. Besonders bevorzugt wird das erfindungsgemäße Verfahren zum Nachweis verstreuter Tumorzellen epidermalen Ursprungs im Knochenmark verwendet. Dabei kann das Knochenmark aus dem Beckenkammknochen entnommen werden. In der Probe werden dann vorzugsweise mononukleäre Zellen einschließlich Tumorzellen angereichert. Diese Anreicherung kann nach bekannten Methoden beispielsweise durch Dichtegradienten-Zentrifugation, z.B. Ficoll, erfolgen, wobei eine Abtrennung von Erythrozyten und Granulozyten stattfindet.

Die zu testende Probe enthält vorzugsweise mindestens 10⁶ Zellen, um einen zuverlässigen Nachweis seltener Zellen zu ermöglichen. Besonders bevorzugt enthält die Probe 10⁶ bis 10⁹, insbesondere 5x10⁶ bis 5x10⁷ Zellen.

Gemäß Schritt (a) wird die Probe mit mindestens zwei verschiedenen, gegen die nachzuweisenden Zellen gerichteten Bindemolekülen in Kontakt gebracht. Die Bindemoleküle sind vorzugsweise Antikörper oder Antikörperfragmente, insbesondere monoklonale Antikörper oder Antikörperfragmente. Darüber hinaus können jedoch auch Liganden von in den nachzuweisenden Zellen spezifisch vorhandenen Rezeptoren, z.B. dem uPA-Rezeptor, verwendet werden. Beispiele solcher Liganden sind lineare oder/und zyklische Peptide oder Peptidmimetika, die eine Fluoreszenzmarkierung tragen können.

Das Inkontaktbringen der Probe mit den fluoreszenzmarkierten Bindemolekülen erfolgt vorzugsweise nach Fixierung der Zellen auf eine Festphase. Diese Fixierung kann nach bekannten Methoden, z:B: mit Formaldehyd oder Glutardialdehyd, erfolgen. Als Festphase kann beispielsweise ein Objektträger verwendet werden.

Sofern erforderlich, können die in der zu testenden Probe vorliegende Zellen unter Verwendung eines Detergens, z.B. eines Saponins, permeabilisiert werden. Auf diese Weise können die Bindemoleküle auch an intrazellulär lokalisierte Determinanten binden.

Beim Nachweis von Tumorzellen sind die Bindemoleküle gegen Determinanten gerichtet, die in der zu testenden Probe nur oder in erhöhter Konzentration in Tumorzellen, aber nicht oder nur in geringer Konzentration in normalen Zellen vorkommen. Vorzugsweise wird als erste Determinante eine Struktur aus dem Inneren der Zellen, z.B. ein Cytokeratin, ausgewählt. Cytokeratine sind für Epithelzellen spezifische Bestandteile des Cytoskeletts und werden in mononukleären Blut- oder Knochenmarkszellen, die mesenchymalen Ursprungs sind, nicht exprimiert. Die Anwesenheit von Cytokeratinen in Zellen, die aus Blut- und Knochenmark entnommen wurden, weist somit auf das Vorhandensein epithelialer Tumorzellen hin. Beispiele für geeignete Anti-Cytokeratin-Antikörper sind der Antikörper A45B/B3 (Micromet GmbH, München, Deutschland) oder der Antikörper CK2 (Boehringer Mannheim GmbH, Mannheim, Deutschland). Weitere gegen intrazelluläre tumorassoziierte Antigene gerichteten Nachweisantikörper sind bekannt und kommerziell von verschiedenen Firmen erhältlich.

Als zweite Determinante dient in jedem Fall der Urokinaserezeptor (uPAR). Der Nachweis dieses Rezeptors kann beispielsweise unter Verwendung von Anti-uPAR-Antikörpern wie etwa IID7 und IIIF10 (Luther et al., Am. J. Path. 150 (1997), 1231-1244) erfolgen. Vorzugsweise werden solche Anti-uPAR-Antikörper ausgewählt, die eine mindestens vergleichbare Affinität für einen Tumorzellenspezifischen uPAR aufweisen, wie für einen uPAR aus "normalen" Zellen. Beispiele für solche auch Tumorzellen mit hoher Affinität bindende Anti-uPAR-Antikörper sind Antikörper, die das Epitop 52-60 von uPAR erkennen wie etwa der bereits oben genannte Antikörper IIIF10.

Andere Anti-uPAR-Antikörper erkennen hingegen uPAR auf Tumorzellen oftmals nur schlecht.

Andererseits kann der Nachweis von uPAR auch mit Fluoreszenzmarkierten Rezeptorliganden, z.B. Urokinase, Urokinasefragmenten oder Urokinase-Peptiden erfolgen. Derartige Nachweismethoden sind beispielsweise von Chucholowski et al. (Fibrinolysis 6, Suppl. 4 (1992), 95-102), Ciccocioppo et al. (J. Histochem. Cytochem. 45 (1997), 1307-1313) und Luther et al. (Am. J. Pat. 150 (1997), 1231-1242) beschrieben.

Im erfindungsgemäßen Verfahren werden mindestens zwei verschiedene Fluoreszenzmarkierungsgruppen verwendet. Günstigerweise werden solche Fluoreszenzmarkierungsgruppen eingesetzt, die voneinander unterscheidbare Emissionsspektren besitzen (z.B. rot/grün). Beispiele für geeignete Fluoreszenzfarbstoffe sind Fluorescein und Derivate davon, Phycoerythrin, Rhodamin, TRITC-Amine, Texas Red^{®}-Amine, CY3 und CY5 sowie Alexa^{®} 488 und Alexa^{®} 568 (Molecular Probes). Die Fluoreszenzfarbstoffe können direkt, z.B. kovalent, mit den primären, für die nachzuweisende Zellen spezifischen Bindemolekülen konjugiert werden. In diesem Fall spricht man von einer Direktmarkierung. Andererseits können die Fluoreszenzfarbstoffe an sekundäre Bindemoleküle konjugiert werden, welche ihrerseits gegen die primären Bindemoleküle gerichtet sind. Indiesem Fall spricht man von einer Indirektmarkierung. Beide Markierungsmethoden, bzw. Kombinationen davon können beim erfindungsgemäßen Verfahren verwendet werden.

Die verschiedenen Bindemoleküle können mit der Zelle sequenziell oder parallel inkubiert werden. Eine parallele Inkubation mit mehreren Bindemolekülen (primäre Bindemoleküle und gegebenenfalls sekundäre Bindemoleküle bei Indirektmarkierung) führt zu einer erheblichen Zeitersparnis.

Die Auswertung der Probe erfolgt durch Bestimmung der Fluoreszenz nach Anregung der Fluoreszenzmarkierungsgruppen. Besonders bevorzugt wird hierfür ein konfokales Laser-Scanning-Mikroskop oder ein Fluoreszenzmikroskop verwendet, welches eine Auswertung der Probe durch parallele oder/und sequenzielle Bestimmung der verschiedenen Fluoreszenzmarkierungsgruppen ermöglicht.

Die erfindungsgemäße Doppelfluoreszenz-Markierungstechnik ermöglicht darüber hinaus eine Charakterisierung der durch Reaktion mit den Bindemolekülen als positiv identifizierten Zellen. Diese Charakterisierung kann eine ortsspezifische oder/und quantitative Auswertung der Markierung umfassen. So kann ein "Scannen" von einzelnen Zellen durch Bestimmung der Markierung in mehreren, z.B. 10 bis 50, Schnittebenen durch die Zelle in Abständen von beispielsweise 0,1 bis 1 µm erfolgen. Zusätzlich kann anhand einer Standardkurve, die durch die Messung von Mikropartikeln definierter Größe und definierter Menge an Fluoreszenzfarbstoff erstellt wurde, auch eine quantitative Bestimmung der mit den Bindemolekülen reagierenden Determinanten in der Zelle durchgeführt werden.

Das erfindungsgemäße Verfahren erlaubt die Gewinnung wertvoller diagnostischer Daten an Tumorpatienten und ermöglicht daher eine sensitive Prognosestellung für den Patienten nach Operation eines Primärtumors.

Schließlich betrifft die Erfindung die Verwendung eines Reagenzienkits zum quantitativen Nachweis von Zellen in einer biologischen Probe in vitro in dem oben genannten Verfahren. Das Reagenzienkit umfasst
(a) ein erstes, die nachzuweisenden Zellen erkennendes Bindemolekül und eine erste an das Bindemolekül direkt konjugierte Fluoreszenz-Markierungsgruppe, wobei das Bindemolekül für den Urokinaserezeptor spezifisch ist,
(b) ein zweites, die nachzuweisenden Zellen erkennendes Bindemolekül und eine zweite an das Bindemolekül direkt konjugierte Fluoreszenz-Markierungsgruppe, wobei das erste und das zweite Bindemolekül und die erste und die zweite Fluoreszenz-Markierungsgruppe verschieden sind und
(c) Mittel zur Fixierung von Zellen auf einer Festphase.

Überraschenderweise wurde festgestellt, daß uPAR-Antikörper, die gegen das Epitop 52-60 von uPAR gerichtet sind, einen uPAR mit einer in Tumorzellen vorkommenden Glykostruktur erkennen, d.h. mit mindestens vergleichbarer Affinität an einen von Tumorzellen exprimierten uPAR wie an einen von Normalzellen exprimierten uPAR binden. Andere Anti-uPAR-Antikörper z.B. HD13.1 (Todd et al., CD87 workshop panel report. In: Kishimoto T. et. al., Hrsg., Leucocyte Typing VI, New York & London, Garland Publishing, Inc. 1997; 1016-1020) besitzen hingegen nur eine geringe Affinität für uPAR aus Tumorzellen.

Zur Herstellung eines gegen uPAR auf Tumorzellen gerichteten diagnostischen oder therapeutischen Mittels ist somit ein Antikörper oder ein antigenbindendes Fragment davon geeignet (vorzugsweise ein monoklonaler Antikörper oder ein antigenbindendes Fragments davon), der gegen das Epitop 52 bis 60 von uPAR gerichtet ist. Derartige Antikörper wie etwa der bekannte monoklonale Antikörper IIIF10 (Luther et. al. (1997), supra) oder Antikörper mit äquivalenter

Bindespezifität, wie etwa chimärisierte oder humanisierte Antikörper oder entsprechende rekombinante oder proteolytische Antikörperfragmente, z. B. einzelkettige Antikörper-Fragmente, erkennen einen von Tumorzellen exprimierten uPAR mit einer für diagnostische und therapeutische Zwecke ausreichenden Affinität.

Weiterhin wurde überraschend festgestellt, daß derartige Antikörper oder Fragmente davon als-diagnostisches Mittel zur Prognose des Verlaufs bei malignen Erkrankungen, insbesondere bei Tumoren, z.B. Mammakarzinomen, eingesetzt werden können. In Tumorproben von über 200 untersuchten Mamakarzinompatientinnen wurde gefunden, daß die Bindung des Antikörpers IIIF10 oder eines entsprechenden Antikörpers mit äquivalenter Bindefähigkeit eine signifikante prognostische Relevanz für den Krankheitsverlauf, d.h. Rezidivfreiheit bzw. Versterben aufweist. Dabei bedeuten hohe Antigenwerte eine kürzere Rezidivfreiheit bzw. früheres Versterben. Mit Antikörpern, die gegen andere Regionen von uPAR gerichtet sind, konnte eine derartige prognostische Signifikanz nicht gefunden werden.

Aufgrund der hohen Affinität zu Tumor-uPAR eignen sich diese Antikörper oder Fragmente davon auch als diagnostische Mittel zum Nachweis von Tumorzellen in einer biologischen Proben, insbesondere zum Nachweis von verstreuten Tumorzellen in Knochenmark. Derartige Nachweisverfahren können beispielsweise als ELISA oder als - zuvor im Detail erläutertes - Doppelfluoreszenz-Nachweisverfahren durchgeführt werden.

Darüberhinaus sind Antikörper, die gegen das Epitop 52 bis 60 von uPAR gerichtet sind, oder deren Fragmente zur Herstellung eines therapeutischen Mittels geeignet, welches beispielsweise eine selektive Funktionsblockierung bei Tumorzellen bewirken kann. Darüberhinaus können die Antikörper oder deren Fragmente in Form von Konjugaten mit einer cytotoxischen Gruppe zur Wachstumshemmung oder Abtötung von Tumorzellen eingesetzt werden. Beispiele für geeignete cytotoxische Gruppen sind radioaktive Gruppen, Toxine und Zellwachstumsinhibitoren. Für therapeutische Zwecke werden vorzugsweise chimäre Antikörper mit humanisierten konstanten Domänen eingesetzt, deren Herstellung beispielsweise in EP-B-O 120 694 beschrieben ist.

Offenbart werden ebenfalls rekombinante Nukleinsäuren, die für ein Polypeptid mit Antikörpereigenschaften kodieren und die CDR3-VH-Sequenz oder/und die CDR3-VL-Sequenz des Antikörpers IIIF10 umfassen. Die CDR3-Region der VH-cDNA ist in SEQ ID NO. 1/2 von Nukleotid 295 bis 321 (entsprechend Aminosäure 99 bis 107) dargestellt. Die CDR3-Region der VL-cDNA ist in SEQ ID NO. 3/4 von Nukleotid 265 bis 291 (Aminosäure 89 bis 97) dargestellt. Weiterhin enthalten die Nukleinsäuren vorzugsweise die für die CDR1- und/oder CDR2-Regionen kodie-renden Abschnitte der VH- oder/und VL-cDNA. Die Sequenzen für die CDR1-VH-Region sind in SEQ ID NO. 1/2 von Nukleotid 91 bis 105 (entsprechend Aminosäure 31 bis 35, d.h. SYDIN) angegeben. In SEQ ID NO. 3/4 reicht die CDR1-Region der VL-cDNA von Nukleotid 70 bis 102 (entsprechend Aminosäure 24 bis 34, d.h. KAS...TVA). Die CDR2-Region der VH-cDNA reicht von Nukleotid 148 bis 198 (Aminosäure 50 bis 66, d.h. WIF...FKD) in SEQ ID NO. 1/2. Die CDR2-Region der VL-cDNA reicht von Nukleotid 148 bis 168 (entsprechend Aminosäure 50 bis 56, d.h. LASNRHT) in SEQ ID NO. 3/4.

Insbesondere werden rekombinante Nukleinsäuren offenbart, die für ein Polypeptid mit Antikörpereigenschaften kodieren, umfassend
(a) eine CDR3-VH-Sequenz kodierend für die Aminosäuresequenz (I):
   D G S M G G F D Y
   oder/und
(b) eine CDR-3-VL-Sequenz kodierend für die Aminosäuresequenz (II):
   L Q H W N Y P Y T

Weiterhin betrifft die Erfindung rekombinante Polypeptide mit Antikörpereigenschaften umfassend
(a) eine CDR3-VH-Aminosäuresequenz (I):
   D G S M G G F D Y
   oder/und
(b) eine CDR-3-VL-Aminosäuresequenz (II):
   L Q H W N Y P Y T

Die rekombinanten Nukleinsäuren und Polypeptide enthalten vorzugsweise die CDR3-Regionen sowohl der VH- als auch der VL-Sequenz. Besonders bevorzugt sind die rekombinanten Polypeptide einzelkettige Antikörper, z.B. scFv-Antikörperfragmente. Bei den rekombinanten Polypeptiden sind die nicht unmittelbar für die Antigenbindung verantwortlichen Framework-Domänen vorzugsweise durch entsprechende humane Sequenzen ersetzt, so daß humanisierte Antikörperfragmente entstehen. Die erfindungsgemäßen rekombinanten Polypeptide können mit Effektorgruppen, d.h. cytotoxischen Gruppen für therapeutische Zwecke oder/und Nachweisgruppen für ein Tumorimaging gekoppelt sein.

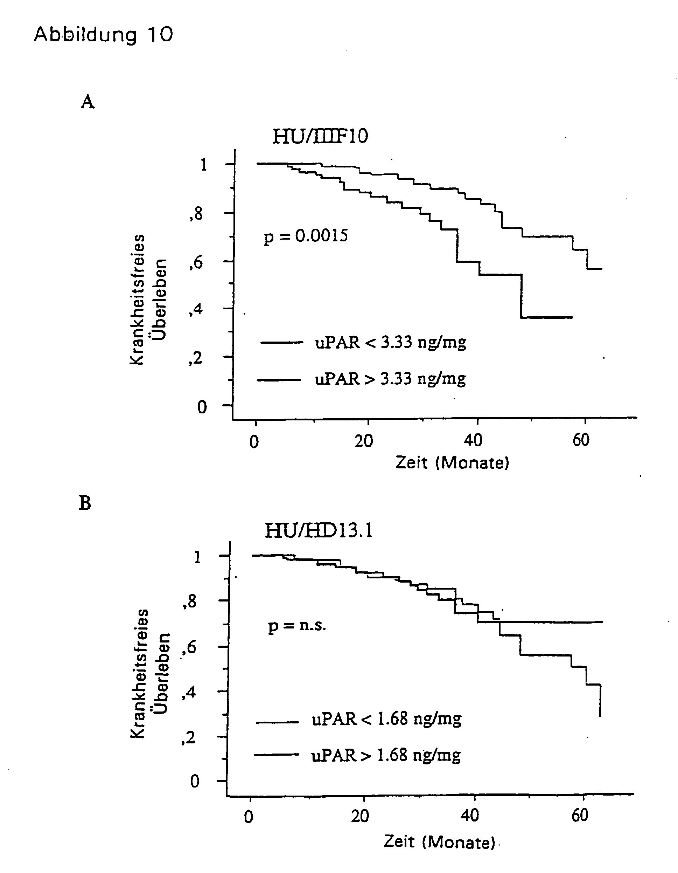

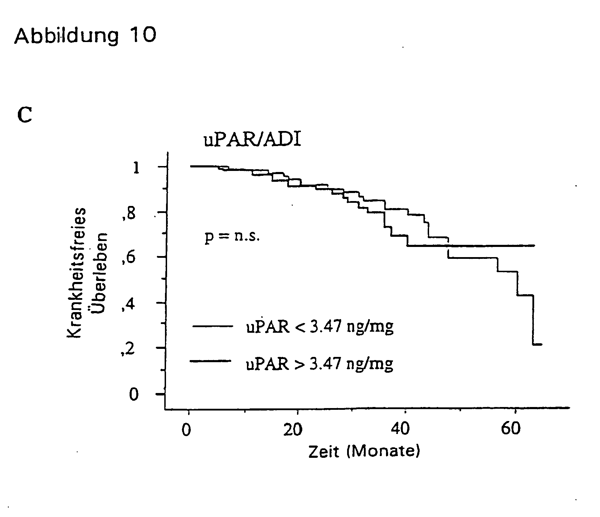

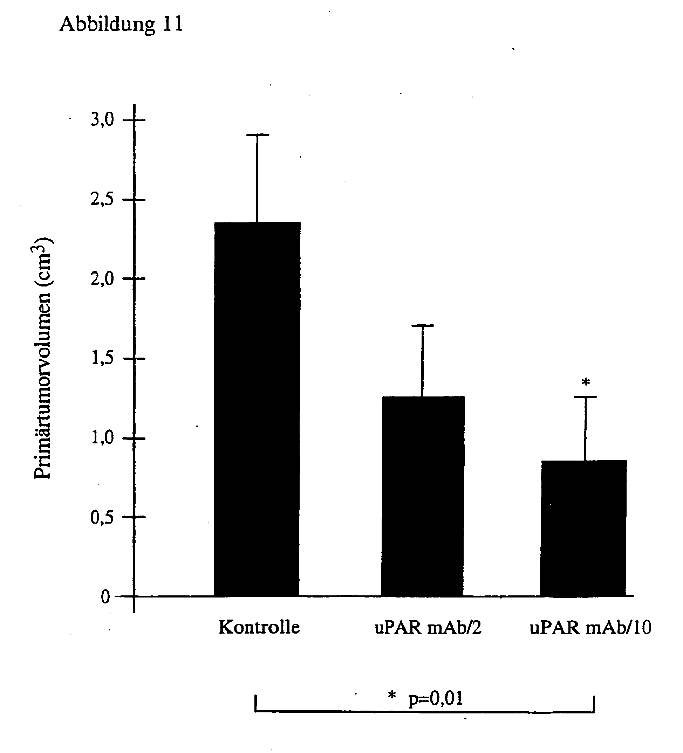

Weiterhin wird die Erfindung durch die nachfolgenden Abbildungen und Beispiele erläutert. Es zeigen:

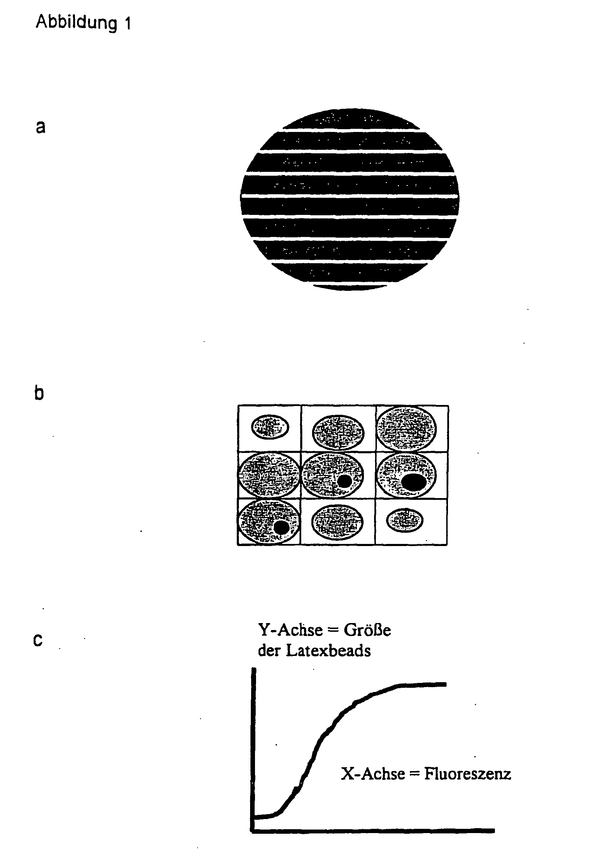

- Abbildung 1:: Eine schematische Darstellung des "Scannens" einer Zelle im Lasermikroskop.
a) Von einer ca. 15 µm großen Tumorzelle werden insgesamt 30 Serienschnitte im Abstand von jeweils 0,5 µm angelegt.
b) Die Messung der Fluoreszenz wird in jeder Schnittebene durchgeführt und dann alle Fluoreszeniwerte addiert.
c) Anhand einer Standardkurve (Latex-Mikropartikel mit definierter Fluorochrommenge) erfolgt eine Berechnung der Gesamtfluoreszenz.

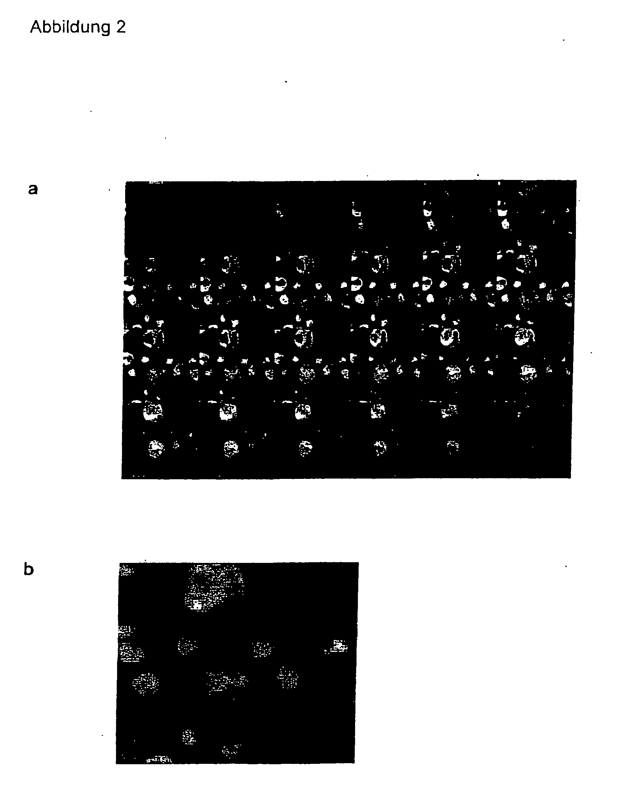

- Abbildung 2:: das Ergebnis der Fluoreszenzanfärbung einer Tumorzelle mit dem Anti-Cytokeratin-Antikörper A45 B/B3 und Alexa 488 als Fluoreszenzfarbstoff.
a) Die Bildsequenz zeigt 24 Aufnahmen einer Scanprozedur, bei der eine ca. 12 µm große Mammakarzinomzelle (ZR75) in Schnittebenen von jeweils 0,5 µm Abstand vermessen wurde.
b) Es ist eine "extended focus" Aufnahme gezeigt, bei der die Gesamtintensität des gesamten Scans (a) auf eine einzige Bildebene projiziert wurde.
- Abbildung 3:: das Ergebnis einer indirekten Fluoreszenzanfärbung mit A45B/B3 als Primärantikörper und einem mit Alexa 488 konjugierten Sekundärantikörper (Vergrößerung x63),
a) Transmissionsbild
b) eine Cytokeratin-positive Zelle im Knochenmarkausstrich einer Patientin mit Mammakarzinom.
- Abbildung 4:: das Ergebnis einer direkten Fluoreszenzanfärbung mit einem Konjugat des Antkörpers A45B/B3 und dem Fluoreszenzfarbstoff Alexa 488 (Vergrößerung x63),
a) Transmissionsbild
b) Cytokeratinnachweis in einer Mischpräparation aus MCF7-Tumorzellen und peripheren Blutlymphozyten (1:20)
- Abbildung 5:: das Ergebnis einer direkten Fluoreszenzanfärbung mit einem Konjugat des Anti uPAR-Antikörpers IIIF10 und dem Fluoreszenzfarbstoff Alexa 568 (Vergrößerung x63),
a) Transmissionsbild
b) uPA-Rezeptornachweis in einer-Mischpräparation aus MCF 7-Tumorzellen und peripheren Blutlymphozyten (1:20)
- Abbildung 6:: das Ergebnis einer direkten Doppelfluoreszenzanfärbung mit den Konjugaten A45 B/B3-Alexa 488 (Anti-Cytokeratin) und IIIF10-Alexa 568 (Anti-uPAR),
a) Transmissionsbild
b) Cytokeratinnachweis
c) uPA-Rezeptornachweis
- Abbildung 7:: das Ergebnis der Vermessung einer Tumorzelle im Knochenmark (Vergrößerung x 63),
a) Transmissionsbild (Nomarski-Optik)
b) Reaktion der Zelle mit einem Konjugat aus Alexa 488 und einem Anti-Cytokeratinantikörper
c) Reaktion der Zelle mit einem Konjugat aus Alexa 568 und einem uPAR-Antikörper. Der Zellkern ist nicht gefärbt. Die Reaktion des Anti-uPAR-Antikörpers beschränkt sich hauptsächlich auf die Zellmembran. Rechts unten ist eine uPAR-positive Knochenmarkszelle, welche negativ für Cytokeratin ist, dargestellt. Alle anderen Zellen sind uPAR-negativ.

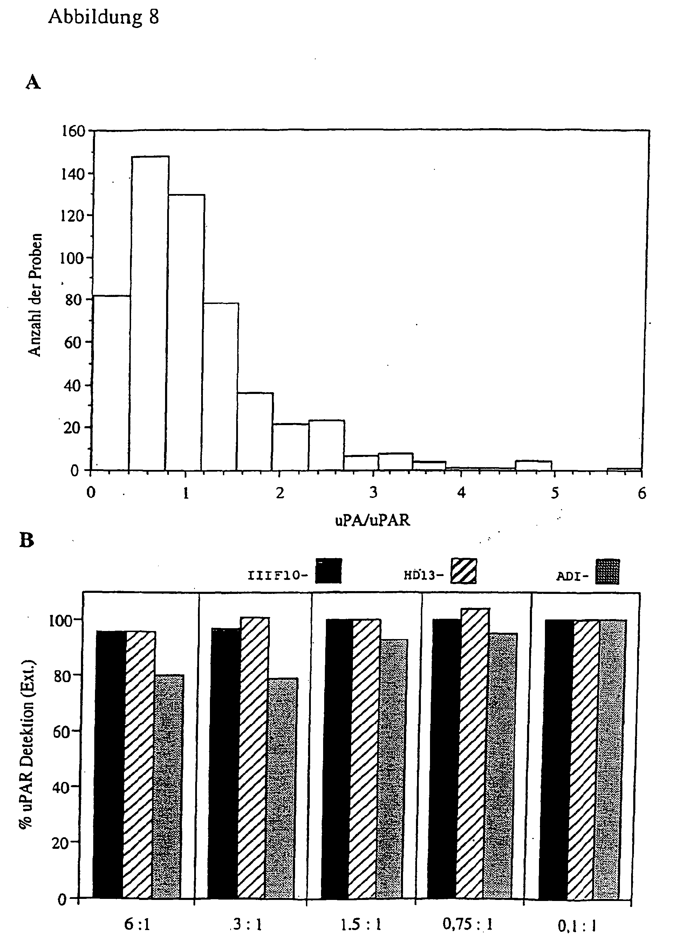

- Abbildung 8:: den Einfluß von uPA auf die uPAR Bestimmung
a) das UPA/uPAR-Verhältnis in Tumorextrakten von 599 Mammakarzinompatientinnen,
b) die Bestimmung von uPAR in Gegenwart unterschiedlicher uPA-Mengen,

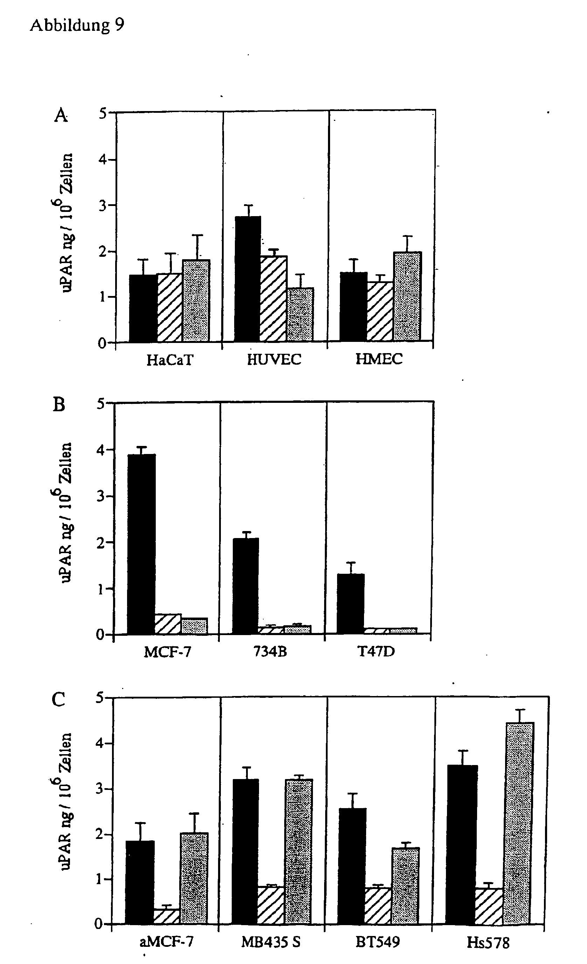

- Abbildung 9:: den uPAR-Antigengehalt in verschiedenen Zellen bestimmt durch unterschiedliche Testverfahren:
IIIF10/HU277 schwarz, HD13.1/HU277: dunkelgrau,
ADI hellgrau
a) normale Zellen
b) gut differenzierte Tumorzellen
c) schlecht differenzierte Tumorzellen
- Abbildung 10:: die prognostische Relevanz des uPAR-Antigengehalts bestimmt durch unterschiedliche Testverfahren an 203 Mammakarzinompatientinnen
a) IIIF10/HU277
b) HD13.1/HU277
c) ADI
- Abbildung 11:: die dosisabhängige Inhibierung des Tumorwachstums von humanem Brustkrebs in Nacktmäusen bei Verabreichung des Antikörpers IIIF10.

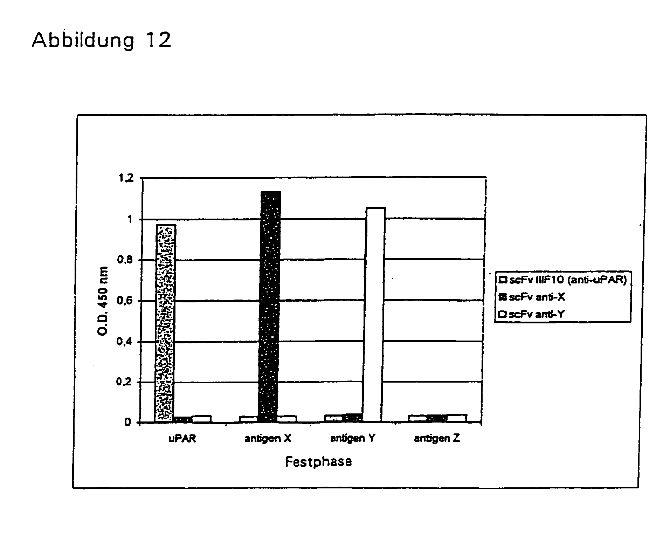

- Abbildung 12:: die Bindung von scFv IIIF10 an immobilisierte Antigene.

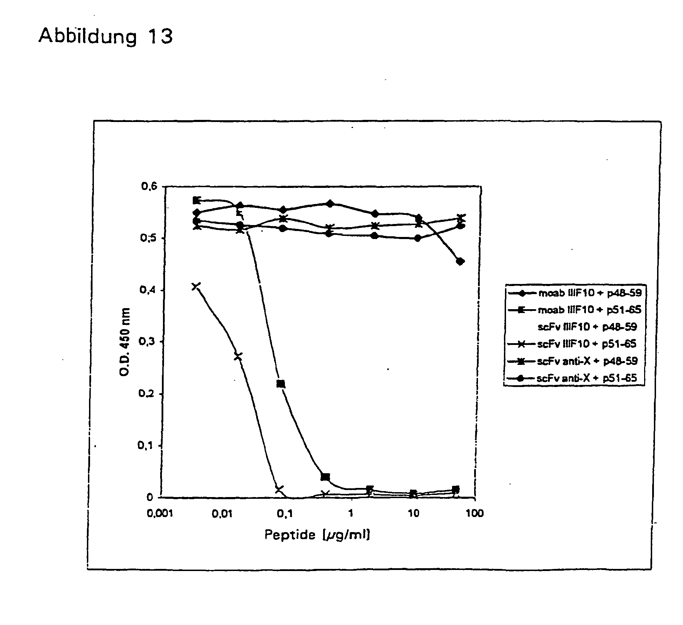

- Abbildung 13:: die Hemmung der Bindung von IIIF10 (monoklonaler Antikörper/moab und scFv) an uPAR durch Peptide.
- SEQ IN NO 1/2:: die Nukleotidsequenz der für die VH-Kette von IIIF10 VH kodierenden cDNA und die korrespondierende Aminosäuresequenz.
- SEQ ID NO 3/4:: die Nukleotidsequenz der für die VL-Kette von IIIF10 kodierenden cDNA und die korrespondierende Aminosäuresequenz.

### Beispiele

### 1. Doppelfluoreszenzbestimmung von Tumorzellen

### 1.1 Material

Der monoklonale Maus-Antikörper A45B/B3 (Kaspar et al., Eur. J. Cancer Clin. Oncol 23, (1987), 137-147) ist gegen die Cytokeratin-Filamente 8, 18 und 19 (CK 8,18,19) gerichtet. Dieser Antikörper wurde mit dem Fluorochrom ALEXA 488 von Molecular Probes direktkonjugiert. Der uPA-Rezeptor wird vom monoklonalen Maus-Antikörper IIIF10 (Luther et al. (1997), supra) (Epitop 52 bis 60) spezifisch detektiert. Als weitere uPA-Rezeptor-Antikörper stehen die monoklonalen Antikörper HD 13.1 und II D7 (Luther et. al. (1997), supra) (Epitop 125 bis 132), sowie der polyklonale Kaninchenantikörper #399R (Stahl et. al., Cancer Res. 54 (1994), 3066-3071) und der Hühnerantikörper HU277 (Magdolen et al., Electrophoresis 16 (1995), 813-816) zur Verfügung. Alle monoklonalen Antikörper gegen den uPA-Rezeptor wurden mit dem Fluoreszenzfarbstoff ALEXA^{®} 568 direktkonjugiert.

**Tabelle 1 Verwendete direktkonjugierte Antikörper**

| **Monoklonaler Antikörper** | **Antigen** | **Direktkonjugiert mit** | **Anregungsbereich im CLSM*** | **Hersteller** |
|---|---|---|---|---|
| mAb II D 7 (Maus) | uPAR, Domäne 2 | ALEXA 568 (^{™}Molecular Probes) | 568 nm | Pathologie Dresden und Frauenklinik München |
| mAb III F 10 (Maus) | uPAR, Domäne 1 | ALEXA 568 (^{™}Molecular Probes) | 568 nm | Pathologie Dresden und Frauenklinik München |
| mAb HD 13.1 (Maus) | uPAR, Domäne 2 + 3 | ALEXA 568 (^{™}Molecular Probes) | 568 nm | Immunologie Heidelberg |
| mAb A45 B/B 3 (Maus) | Cytokeratin 8/9/18 | ALEXA 488 (^{™}Molecular Probes) | 488 nm | Micromet München |

| | | | | |
|---|---|---|---|---|
| (*CLSM=konfokales Laser-Scanning-Mikroskop) | | | | |

### 1.2 Knochenmarkpräparate

Im Operationssaal wird eine Jamshidi-Punktion durchgeführt. Beiderseits werden jeweils 4-6 ml Knochenmark aus den Beckenkammknochen entnommen. Die Anreicherung der Tumorzellen in der Fraktion der mononukleären Zellen erfolgt über einen Ficollgradienten. 8 bis 12 Cytospins (10⁶ Zellen pro Cytospin) werden pro Patient hergestellt. Nach Lufttrocknung werden die Präparate fixiert und permeabilisiert.

### 1.3 Fixierung und Permeabilisierung

1. Fixieren in 4% Paraformaldehyd (PFA) für 30 min.
2. Dreimal in Phosphat-gepufferter Salzlösung/Rinderserumalbumin (PBS/BSA) 1 % waschen.
3. Permeabilisieren in 0,025% Saponin für 45 min.
4. Dreimal in PBS/BSA 1% waschen.

### 1.4 Doppelmarkierung von Cytokeratin-und uPA-Rezeptor

### 1.4.1 Indirekte Methode

1. Inkubation über Nacht mit dem Primär-Maus-Antikörper A 45 B/B3 (Endkonzentration 0,004 mg/ml) in PBS/BSA 1% verdünnt.
2. Dreimal in PBS/BSA 1% waschen.
3. Inkubation mit dem zweiten in PBS/BSA 1% verdünnten Primär-Kaninchen-Antikörper #399 R (Endkonzentration 0,05 mg/ml) für 2 Stunden.
4. Dreimal in PBS/BSA 1% waschen
5. Sekundärantikörper Ziege-Anti-Maus-Alexa 488 (Endkonzentration 0,02 mg/ml) PBS/BSA 1% verdünnt, Inkubationszeit 30 min
6. Dreimal in PBS/BSA 1% waschen
7. Sekundärantikörper Ziege-Anti-Kaninchen-Alexa 568 (Endkonzentration 0,02 mg/ml) PBS/BSA 1% verdünnt, Inkubationszeit 30 min
8. Dreimal in PBS/BSA 1% waschen
9. Eindeckeln mit 5 µl PBS/BSA 1% und mikroskopieren

### 1.4.2 Direkte Methode

1. Inkubation für 1 Stunde mit dem Antikörper A 45 B/B3-Alexa 488 (Endkonzentration 0,0014 mg/ml) in PBS/BSA 1% verdünnt.
2. Dreimal in PBS/BSA 1% waschen.
3. Inkubation über 1 Stunde mit dem Antikörper III F 10-Alexa 568 (Endkonzentraion 0,003 mg/ml) in PBS/BSA 1% verdünnt.
4. Dreimal in PBS/BSA 1% waschen.
5. Eindeckeln mit 5 µl PBS/BSA 1% und mikroskopieren.

### 1.5 Quantifizierung

Die mit dem fluoreszierenden Antikörper reagierenden Antigene werden im konfokalen Laser-Scanning-Mikroskop bei einem Anregungsbereich von 488 nm bzw. 568 nm sichtbar. Durch "Scannen" der Zelle im Lasermikroskop, d.h. durch Durchschichten in 0,5 µm Schritten, werden Tumorzellen in 20 bis 30 Schnittebenen aufgeteilt. Alle Fluoreszenzen werden erfaßt, die Summe dieser Messungen wird errechnet. Anhand einer Standardkurve, die durch die Messung von Latexbeads mit einer definierten Menge an Fluoreszenzfarbstoff zuvor erstellt wurde, ist eine Quantifizierung der Antigene, die mit dem Antikörper reagiert haben, in der Tumorzelle möglich.

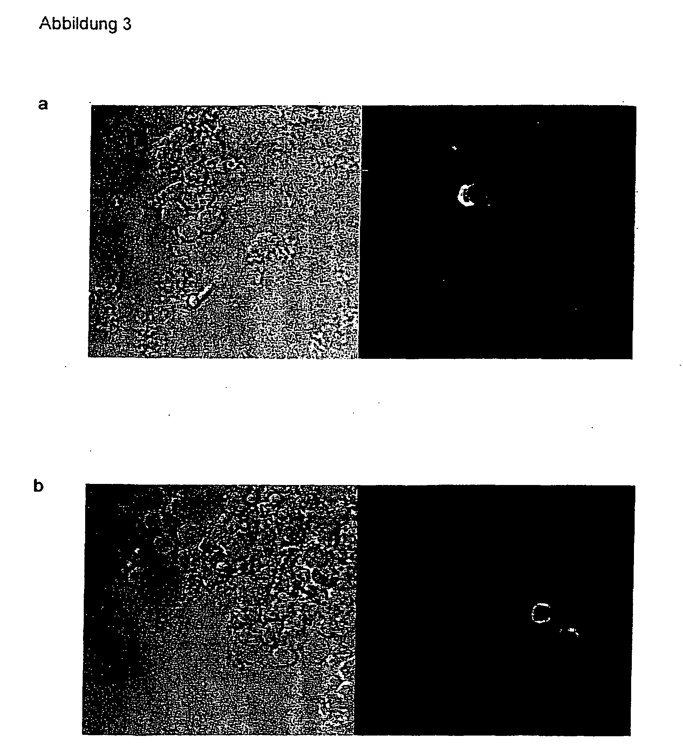

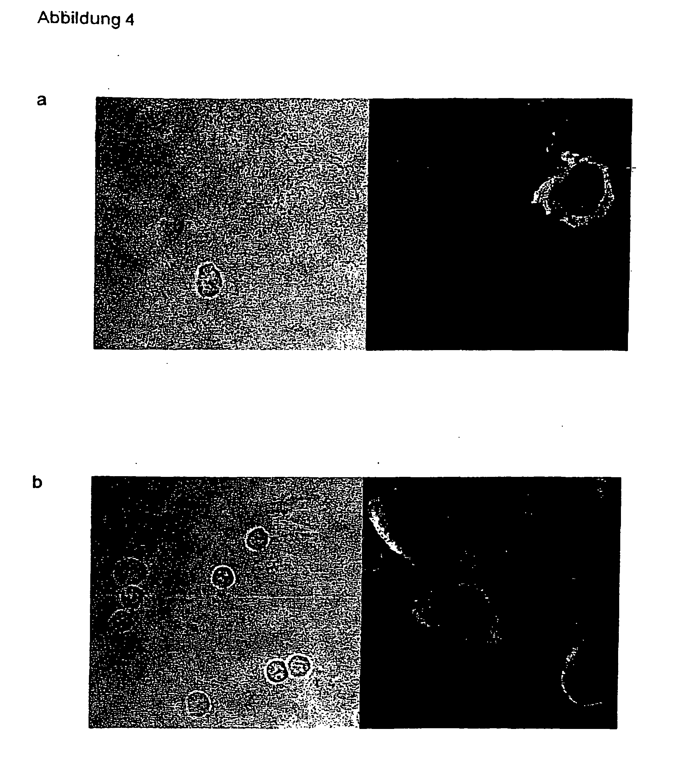

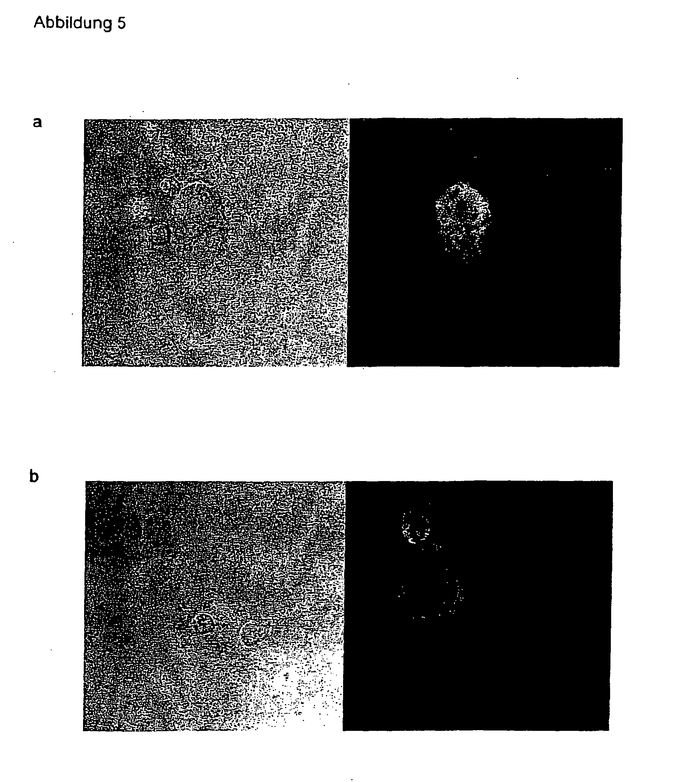

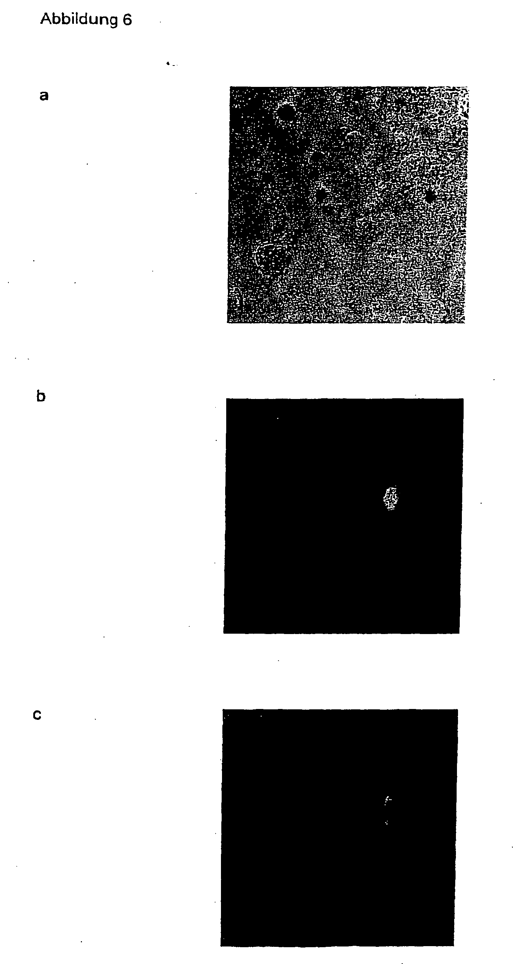

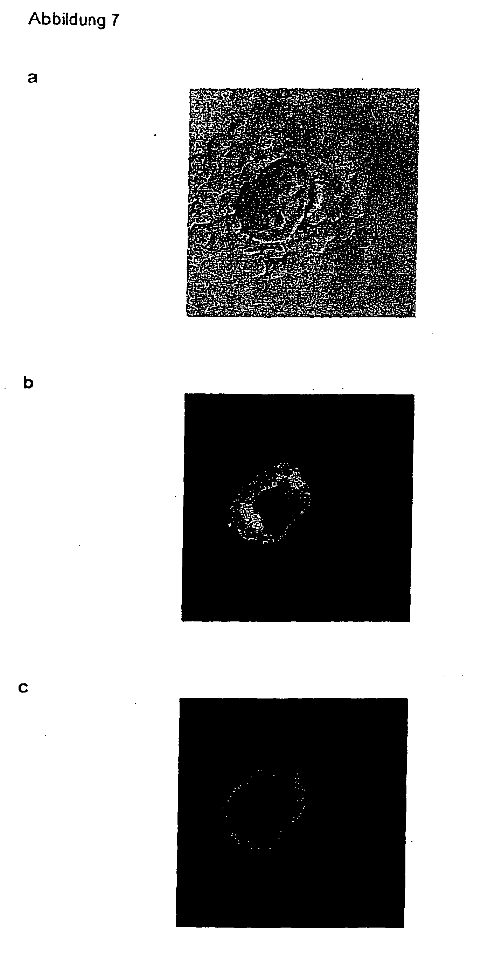

In Abbildung 1 ist das Prinzip der zur Lokalisierung und Quantifizierung der Fluoreszenzmarkierung verwendeten Scanning-Prozedur graphisch dargestellt. Die Abbildungen 2 bis 7 zeigen beispielhafte Ergebnisse für die praktische Anwendung des erfindungsgemäßen Verfahrens.

### 2. Tumorspezifität des monoklonalen Antikörpers IIIF10

Es wurden zwei verschiedene ELISA-Systeme für den Nachweis von uPAR-Antigen entwickelt:
1) Fängerantikörper: polyklonaler Hühnerantikörper HU277 (Magdolen et al. (1995), supra); Nachweisantikörper: Monoklonaler Antikörper IIIF10 (Luther et al. (1997), supra)
2) Fängerantikörper: polyklonaler Hühnerantikörper HU277; monoklonaler Antikörper HD13.1 (Todd et al.(1997), supra).

Diese ELISA-Systeme wurden mit einem kommerziell erhältlichen ELISA (ADI) für uPAR (American Diagnostica Inc. Greenwich, CT, USA) verglichen.

Die getesteten ELISA-Systeme wurden mit rekombinantem, in CHO-Zellen exprimierten, affinitätsgereinigten humanen uPAR (rec-uPAR) aufeinander eingestellt. Alle drei ELISA-Systeme zeigten gegenüber rec-uPAR eine vergleichbare Linearität und Sensitivität.

In fortführenden Untersuchungen konnte gezeigt werden, daß der tatsächliche uPAR-Antigengehalt auf Zellen auch in Anwesenheit eines bis zu sechsfachen Überschusses von uPAR bestimmt werden kann. Die Wiederfindung war > 95% im Falle des IIIF10/HU277- und des HD13.1/HU277-Tests sowie > 80% im Falle des ADI-Tests. Das uPA/uPAR-Verhältnis in 599 analysierten Tumorextrakten ist typischerweise in 95% der Fälle < 3 (Tests mit ADI-UPA- und ADI-uPAR-ELISA). Diese Ergebnisse sind in Abbildung 8 gezeigt.'

Anschließend wurden uPAR-Antigengehalte in Lysaten verschiedener Zelltypen bestimmt. Hier zeigte sich, daß die uPAR-Antigenbestimmung in nichtmalignen Zellen (z.B. Keratinozyten [HaCaT], Endothelzellen aus der Nabelschnur [HUVEC], epithelialen Zellen an der Mamma [HMEC]) vergleichbare Resultate mit allen drei ELISA-Systemen erbrachte. Bei Tumorzellinien ergab sich hingegen ein deutlich anderes Bild. In gut differenzierten Mammakarzinomzellen erkannte lediglich der IIIF10/HU277 ELISA signifikante Mengen an Tumor-assozüertem uPAR, während in schlecht differenzierten Mammakarzinomzellinien der IIIF10/HU277- und der ADI-ELISA vergleichbare Werte ergaben. Der HD13.1/HU277-ELISA detektierte sowohl bei gut als auch bei schlechtdifferenzierten Karzinomzellen zuwenig uPAR. Die Daten sind in Abbildung 9 gezeigt.

### 3. Prognostische Relevanz des monoklonalen Antikörpers IIIF10

In einer klinischen Studie wurde der uPAR-Antigengehalt mit allen drei in Beispiel 2 beschriebenen ELISA-Systemen in Tumorproben von über 200 Mammakarzinompatientinnen bestimmt. Hier zeigte sich, daß die mit dem IIIF10/HU277-ELISA gemessenen Antigenwerte eine signifikante prognostische Relevanz für den Krankheitsverlauf, d.h. für Rezidivfreiheit bzw. Versterben aufweisen. Mit den beiden anderen ELISA-Systemen konnte eine derartige prognostische Relevanz nicht gefunden werden. Die Daten sind in Abbildung 10 gezeigt.

### 4. In vivo Wirkung des monoklonalen Antikörpers IIIF10

4 bis 6 Wochen alten Balb/C/3 Nacktmäusen wurden an der rechten Flanke 6x10⁶ humane Brustkrebszellen MDA-MB231 (Price et al., Cancer Res. 50 (1990), 717-721) in einem Gesamtvolumen von 300 µl injiziert. Vor der Injektion wurden die Krebszellen mit jeweils 200 µg des murinen monoklonalen Antikörpers IIIF10 in PBS, pH 7,4 vermischt. Anschließend wurden die Mäuse alle drei Tage mit monoklonalen Antikörper IIIF10 in einer Dosis von 2 mg/kg Körpergewicht bzw. 10 mg/kg Körpergewicht intraperitoneal in einem Injektionsvolumen von 300 µl behandelt. Das Volumen der in den Mäusen auftretenden Primärtumoren in cm³ wurde nach vier Wochen durch Messung der beiden größten Durchmesser der Tumoren bestimmt. Den Kontrollmäusen wurde PBS pH 7,4 verabreicht, jede Gruppe bestand aus sechs Mäusen.

Die Ergebnisse sind in Abb. 11 gezeigt. Es ist zu erkennen, daß die Verabreichung des Antikörpers das Wachstum von Primärtumoren stark verringerte. Die Inhibition des Wachstums war bei einer Verabreichung von 10 mg/kg Körpergewicht noch deutlicher als bei einer Verbreichung in einer Dosis von 2 mg/kg Körpergewicht ausgeprägt.

### 5. Herstellung von rekombinantem monoklonalem Antikörper IIIF10

mRNA von IIIF10 produzierenden Hybridomzellen wurde angereichert und in cDNA umgeschrieben. Die für die variablen Regionen der schweren (VH) und leichten (VL) Kette kodierenden cDNA-Fragmente wurden durch RT-PCR unter Verwendung genspezifischer Primer amplifiziert. Die VH- und VL-Gensegmente wurden in einen Phagemid-Vektor kloniert, um die Expression der variablen Regionen als einzelkettiger Antikörper (scFv) zu ermöglichen. Die scFv-Moleküle wurden durch Phagendisplay an der Oberfläche filamentöser Phagen als Fusionsprotein mit dem kleinen Phagenhüllprotein pIII präsentiert. Phagen, die eine funktionelle Expression von scFv-FIII10 zeigten, wurden durch spezifische Bindung von uPAR selektiert. Die selektierten Phagen wurden zur Infektion von E.coli Zellen verwendet, was die Produktion und Sekretion von löslichen scFv-Molekülen in das Kulturmedium ermöglichte. Abbildung 12 zeigt die Bindung des scFv-Überstands an einen auf einer Festphase immobilisierten uPAR. Die Bindefähigkeit der Antikörper scFv-Anti-X und scFv-Anti-Y wurde zu Kontrollzwecken mitgeführt.

Um die Bindespezifität weiter zu testen, wurden Peptide eingesetzt, die zur Kartierung des Epitops des Antikörpers IIIF10 verwendet worden waren (Luther et al., J. Pathol 150 (1997), 1231-1244). Wie aus Abbildung 13 ersichtlich ist, kann nur ein Peptid, dessen Sequenz das vollständige IIIF10 Epitop auf uPAR (51-65) umfaßt, die Bindung des monoklonalen Antikörpers und von scFvIIIF10 an uPAR zu verhindern. Ein anderes Peptid mit einem unvollständigen Sequenzepitop (48 bis 59) ist um einen Faktor > 100 weniger wirksam. Keines der Peptide kann die Bindung eines Kontrollantikörpers scFv-Anti-X an sein Zielprotein X verhindern.

Die Nukleotidsequenz der VH-cDNA und die korrespondierende Aminosäuresequenz sind in SEQ ID NO. 1/2 dargestellt. Die Nukleotidsequenz der VL-cDNA und die korrespondierende Aminosäuresequenz sind in SEQ ID NO. 3/4 dargestellt.

## Patentansprüche

1. Verfahren zum quantitativen Nachweis von epithelialen Tumorzellen in einer biologischen Probe in vitro, wobei die biologische Probe die nachzuweisenden Zellen im Bereich von 1:10⁴ bis 1:10⁷ der gesamten in der Probe vorhandenen Zellen enthält, umfassend die Schritte:
(a) Inkontaktbringen einer zu testenden Probe mit mindestens zwei verschiedenen, die nachzuweisenden Zellen erkennenden Bindemolekülen, wobei die Bindemoleküle mit jeweils verschiedenen Fluoreszenzfarbstoffen markiert werden, wobei eines der Bindemoleküle für den Urokinase-Rezeptor spezifisch ist, und
(b) Bestimmen der Fluoreszenzmarkierungen in der auf einer Festphase fixierten Probe.

2. Verfahren nach Anspruch 1,
**dadurch gekennzeichnet,**
**dass** der Nachweis in einer Knochenmarksprobe erfolgt.

3. Verfahren nach einem der Ansprüche 1 bis 2,
**dadurch gekennzeichnet,**
**dass** man als zellspezifische Bindemoleküle Antikörper oder Antikörperfragmente oder/und Rezeptorliganden verwendet.

4. Verfahren nach einem der Ansprüche 2 bis 3,
**dadurch gekennzeichnet,**
**dass** man ein zweites, Cytokeratin-spezifisches Bindemolekül verwendet.

5. Verfahren nach einem der Ansprüche 1 bis 4,
**dadurch gekennzeichnet,**
**dass** die Bindemoleküle indirekt markiert werden.

6. Verfahren nach einem der Ansprüche 1 bis 4,
**dadurch gekennzeichnet,**
**dass** die Bindemoleküle direkt markiert werden.

7. Verfahren nach einem der Ansprüche 1 bis 6,
**dadurch gekennzeichnet,**
**dass** die quantitative Auswertung der Probe durch ein konfokales Laser-Scanning-Mikroskop oder durch ein Fluoreszenzmikroskop erfolgt.

8. Verfahren nach einem der Ansprüche 1 bis 7,
**dadurch gekennzeichnet,**
**dass** die Auswertung der Probe durch parallele oder/und sequentielle Bestimmung der Fluoreszenz der verschiedenen Markierungsgruppen erfolgt.

9. Verfahren nach einem der Ansprüche 1 bis 8, weiterhin umfassend eine Charakterisierung von durch Reaktion mit den Bindemolekülen identifizierten Zellen.

10. Verfahren nach Anspruch 9,
**dadurch gekennzeichnet**
**dass** die Charakterisierung eine ortsspezifische oder/und quantitative Bestimmung der Fluoreszenzmarkierung umfasst.

11. Verwendung eines Reagenzienkits zum quantitativen Nachweis von Zellen in einer biologischen Probe in vitro in einem Verfahren nach einem der Ansprüche 1-10, wobei der Reagenzienkit umfasst:
(a) ein erstes, die nachzuweisenden Zellen erkennendes Bindemolekül und eine erste an das Bindemolekül direkt konjugierte Fluoreszenz-Markierungsgruppe, wobei das Bindemolekül für den Urokinase-Rezeptor spezifisch ist,
(b) ein zweites, die nachzuweisenden Zellen erkennendes Bindemolekül und eine zweite an das Bindemolekül direkt konjugierte Fluoreszenz-Markierungsgruppe, wobei das erste und das zweite Bindemolekül und die erste und die zweite Fluoreszenz-Markierungsgruppe verschieden sind, und
(c) Mittel zur Fixierung von Zellen auf einer Festphase.

12. Verwendung eines Verfahrens nach einem der Ansprüche 1-10 zum Nachweis von Mikrometastasen in biologischen Proben in vitro.

13. Verwendung nach Anspruch 12, wobei ein Reagenzienkit wie in Anspruch 11 beschrieben, eingesetzt wird.

## Claims

1. Method for the quantitative detection of epithelial tumour cells in a biological sample in vitro, wherein the biological sample contains the cells to be detected in a range from 1:10⁴ to 1:10⁷ of the total cells present in the sample comprising the steps:
(a) contacting a sample to be tested with at least two different binding molecules which recognize the cells to be detected, where the binding molecules are each labelled with different fluorescent dyes and one of the binding molecules is specific for the urokinase receptor, and
(b) determining the fluorescent labels in the sample immobilized on a solid phase.

2. Method according to claim 1,
**characterized in that**
the detection is carried out in a bone marrow sample.

3. Method according to one of the claims 1 to 2,
**characterized in that**
antibodies or antibody fragments or/and receptor ligands are used as cell-specific binding molecules.

4. Method according to one of the claims 2 to 3,
**characterized in that**
a second cytokeratin-specific binding molecule is used.

5. Method according to one of the claims 1 to 4,
**characterized in that**
the binding molecules are indirectly labelled.

6. Method according to one of the claims 1 to 4,
**characterized in that**
the binding molecules are directly labelled.

7. Method according to one of the claims 1 to 6,
**characterized in that**
the sample is evaluated quantitatively by a confocal laser scanning microscope or by a fluorescence microscope.

8. Method according to one of the claims 1 to 7,
**characterized in that**
the sample is evaluated by parallel or/and sequential determination of the fluorescence of the different labelling groups.

9. Method according to one of the claims 1 to 8, additionally comprising a characterization of cells identified by the reaction with the binding molecules.

10. Method according to claim 9,
**characterized in that**
the characterization comprises a site-specific or/and quantitative determination of the fluorescent label.

11. Use of a reagent kit for the quantitative detection of cells in a biological sample in vitro in a method according to one of the claims 1 - 10, wherein the reagent kit comprises:
(a) a first binding molecule which recognizes the cells to be detected and a first fluorescent labelling group directly conjugated to the binding molecule, wherein the binding molecule is specific for the urokinase receptor,
(b) a second binding molecule which recognizes the cells to be detected and a second fluorescent labelling group directly conjugated to the binding molecule, wherein the first and the second binding molecule and the first and the second fluorescent labelling group are different, and
(c) means for immobilizing cells on a solid phase.

12. Use of a method according to one of the claims 1 - 10 for detecting micrometastases in biological samples in vitro.

13. Use according to claim 12, wherein a reagent kit as described in claim 11 is used.

## Revendications

1. Procédé de détection quantitative de cellules tumorales épithéliales dans un échantillon biologique *in vitro,* l'échantillon biologique contenant les cellules à détecter dans la plage de 1 :10⁴ à 1 :10⁷ de l'ensemble des cellules présentes dans l'échantillon, comprenant les étapes suivantes :
(a) mise en contact d'un échantillon à tester avec au moins doux molécules de liaison différentes reconnaissant les cellules à détecter, les molécules de liaison étant chacune marquée par des colorants fluorescents différents, l'une des molécules de liaison étant spécifique au récepteur de l'urokinase, et
(b) détermination des marquages fluorescents dans l'échantillon fixé sur une phase solide.

2. Utilisation selon la revendication 1, **caractérisée en ce que** la détection se fait dans un échantillon de moelle osseuse.

3. Utilisation selon l'une des revendications 1 à 2, **caractérisée en ce qu'**on utilise des anticorps ou des fragments d'anticorps et/ou des ligands de récepteurs comme molécules de liaison spécifiques à des cellules.

4. Utilisation selon l'une des revendications 2 à 3, **caractérisée en ce qu'**on utilise une deuxième molécule de liaison spécifique à la cytokératine.

5. Utilisation selon l'une des revendications 1 à 4, **caractérisée en ce que** les molécules de liaison sont marquées indirectement.

6. Utilisation selon l'une des revendications 1 à 4, **caractérisée en ce que** les molécules de liaison sont marquées directement.

7. Procédé selon l'une des revendications 1 à 6, **caractérisé en ce que** l'évaluation quantitative de l'échantillon a lieu avec un microscope laser confocal ou un microscope à fluorescence.

8. Procédé selon l'une des revendications 1 à 7, **caractérisé en ce que** l'évaluation de l'échantillon a lieu par détermination parallèle et/ou séquentielle de la fluorescence des différents groupes marqueurs.

9. Procédé selon l'une des revendications 1 à 8, comprenant en outre une caractérisation par réaction avec des cellules identifiées par les molécules de liaison.

10. Procédé selon la revendication 9, **caractérisé en ce que** la caractérisation comprend une détermination spatialement spécifique et/ou quantitative du marquage par fluorescence.

11. Utilisation d'un kit réactif pour la détection quantitative de cellules dans un échantillon biologique *in vitro* dans un procédé selon l'une des revendications 1 à 10, dans laquelle le kit réactif comprend :
(a) une première molécule de liaison reconnaissant les cellules à détecter et un premier groupe marqueur par fluorescence conjugué directement à la molécule de liaison, la molécule de liaison étant spécifique au récepteur de l'urokinase,
(b) une deuxième molécule de liaison reconnaissant les cellules à détecter et un deuxième groupe marqueur par fluorescence conjugué directement à la molécule de liaison, dans lequel la première et la deuxième molécule de liaison et le premier et le deuxième groupe marqueur par fluorescence étant différents, et
(c) un moyen de fixation des cellules sur une phase solide.

12. Utilisation d'un procédé selon l'une des revendications 1 à 10 pour la détection de micrométastases dans des échantillons biologiques *in vitro.*

13. Utilisation selon la revendication 12, dans laquelle un kit réactif, tel que décrit dans la revendication 11, est utilisé.


### SEQUENZPROTOKOLL

<110> Wilex Biotechnology GmbH
<120> Diagnostischer und therapeutischer Einsatz von Antikörpern gegen den Urokinase-Rezeptor
<130> 19116PEP
<140>
   <141>
<160> 2
<170> PatentIn Ver. 2.1
<210> 1
   <211> 354
   <212> DNA
   <213> Künstliche Sequenz
<220>
   <223> Beschreibung der künstlichen Sequenz: Phagensequenz
<220>
   <221> CDS
   <222> (1)..(354)
<400> 1
   cag gtg caa ctg cag cag tca gga cct gag ttg gtg aag cct ggg gct     48
   Gln Val Gln Leu Gln Gln Ser Gly Pro Glu Leu Val Lys Pro Gly Ala
    1               5                  10                  15
   tta gtg aag ata tcc tgc aag gct tct ggt tac agt ttc aca agc tac     96
   Leu Val Lys Ile Ser Cys Lys Ala Ser Gly Tyr Ser Phe Thr Ser Tyr
               20                  25                  30
   gat ata aat tgg gtg aag cgg agg cct gga cag gga ctt gag tgg att     144
   Asp Ile Asn Trp Val Lys Arg Arg Pro Gly Gln Gly Leu Glu Trp Ile
           35                  40                  45
   gga tgg att ttt cct gga gat ggt agt acc aat tac aat gag aaa ttc     192
   Gly Trp Ile Phe Pro Gly Asp Gly Ser Thr Asn Tyr Asn Glu Lys Phe
       50                  55                  60
   aag gac aag gcc aca ctg act gct gac aaa tcc tcc agc aca gcc tac     240
   Lys Asp Lys Ala Thr Leu Thr Ala Asp Lys Ser Ser Ser Thr Ala Tyr
   65                  70                  75                  80
   atg cag ctc aac agc ctg act tct gag aac tct gca gtc tat ttc tgt     288
   Met Gln Leu Asn Ser Leu Thr Ser Glu Asn Ser Ala Val Tyr Phe Cys
                   85                  90                  95
   gca aga gat gga agt atg ggg ggg ttt gac tac tgg ggc caa ggg acc     336
   Ala Arg Asp Gly Ser Met Gly Gly Phe Asp Tyr Trp Gly Gln Gly Thr
               100                 105                 110
   acg gtc acc gtc tcc tca                                             354
   Thr Val Thr Val Ser Ser
           115
<210> 2
   <211> 118
   <212> PRT
   <213> Künstliche Sequenz
   <223> Beschreibung der künstlichen Sequenz:
   Phagensequenz
<400> 2
   Gln Val Gln Leu Gln Gln Ser Gly Pro Glu Leu Val Lys Pro Gly Ala
    1               5                  10                  15
   Leu Val Lys Ile Ser Cys Lys Ala Ser Gly Tyr Ser Phe Thr Ser Tyr
               20                  25                  30
   Asp Ile Asn Trp Val Lys Arg Arg Pro Gly Gln Gly Leu Glu Trp Ile
           35                  40                  45
   Gly Trp Ile Phe Pro Gly Asp Gly Ser Thr Asn Tyr Asn Glu Lys Phe
       50                  55                  60
   Lys Asp Lys Ala Thr Leu Thr Ala Asp Lys Ser Ser Ser Thr Ala Tyr
   65                  70                  75                  80
   Met Gln Leu Asn Ser Leu Thr Ser Glu Asn Ser Ala Val Tyr Phe Cys
                   85                  90                  95
   Ala Arg Asp Gly Ser Met Gly Gly Phe Asp Tyr Trp Gly Gln Gly Thr
               100                 105                 110
   Thr Val Thr Val Ser Ser
           115
<210> 3
   <211> 324
   <212> DNA
   <213> Künstliche Sequenz
<220>
   <221> CDS
   <222> (1)..(324)
<220>
   <223> Beschreibung der künstlichen Sequenz:
   Phagensequenz
<400> 3
   gat gtt ttg atg acc caa act cca aaa ttc atg tcc aca tca gta gga       48
   Asp Val Leu Met Thr Gln Thr Pro Lys Phe Met Ser Thr Ser Val Gly
   1               5                   10                  15
   gac agg gtc agc atc acc tgc aag gcc agt cag aat gtt cgt act act       96
   Asp Arg Val Ser Ile Thr Cys Lys Ala Ser Gln Asn Val Arg Thr Thr
               20                  25                  30
   gta gcc tgg tat caa gag aaa cca ggg cag tct cct aaa gca ctg att      144
   Val Ala Trp Tyr Gln Glu Lys Pro Gly Gln Ser Pro Lys Ala Leu Ile
           35                  40                  45
   tac ttg gca tcc aac cgg cac act gga gtc cct gat cgc ttc aca ggc      192
   Tyr Leu Ala Ser Asn Arg His Thr Gly Val Pro Asp Arg Phe Thr Gly
       50                  55                  60
   agt gga tct gga aca gat ttc act ctc acc att agc aat gtg caa tct      240
   Ser Gly Ser Gly Thr Asp Phe Thr Leu Thr Ile Ser Asn Val Gln Ser
   65                  70                  75                  80
   gaa gac ctg gca gat tat ttc tgt ctg caa cat tgg aat tat ccg tac      288
   Glu Asp Leu Ala Asp Tyr Phe Cys Leu Gln His Trp Asn Tyr Pro Tyr
                   85                  90                  95
   acg ttc gga ggg ggc acc aag ctg gaa atc aaa cgg                      324
   Thr Phe Gly Gly Gly Thr Lys Leu Glu Ile Lys Arg
               100                 105
<210> 4
   <211> 108
   <212> PRT
   <213> Künstliche Sequenz
   <223> Beschreibung der künstlichen Sequenz:
   Phagensequenz
<400> 4
   Asp Val Leu Met Thr Gln Thr Pro Lys Phe Met Ser Thr Ser Val Gly
    1               5                  10                  15
   Asp Arg Val Ser Ile Thr Cys Lys Ala Ser Gln Asn Val Arg Thr Thr
                20                  25                  30
   Val Ala Trp Tyr Gln Glu Lys Pro Gly Gln Ser Pro Lys Ala Leu Ile
            35                  40                  45
   Tyr Leu Ala Ser Asn Arg His Thr Gly Val Pro Asp Arg Phe Thr Gly
        50                  55                  60
   Ser Gly Ser Gly Thr Asp Phe Thr Leu Thr Ile Ser Asn Val Gln Ser
    65                  70                  75                  80
   Glu Asp Leu Ala Asp Tyr Phe Cys Leu Gln His Trp Asn Tyr Pro Tyr
                    85                  90                  95
   Thr Phe Gly Gly Gly Thr Lys Leu Glu Ile Lys Arg
               100                 105